(19) **Europäisches Patentamt**

**European Patent Office**

**Office européen des brevets**

(11) **EP 1 438 336 B1**

(12) **EUROPEAN PATENT SPECIFICATION**

(45) Date of publication and mention
of the grant of the patent:
**14.06.2006 Bulletin 2006/24**

(51) Int Cl.:
*C07K 14/59* *(2006.01)*    *A61K 38/24* *(2006.01)*

(21) Application number: **02774712.0**

(22) Date of filing: **15.10.2002**

(86) International application number:
**PCT/EP2002/011501**

(87) International publication number:
**WO 2003/035686 (01.05.2003 Gazette 2003/18)**

(54) **COMPOSITIONS OF FSH WITH HIGH SIALYLATION DEGREE AND THEIR USE FOR THE PREPARATION OF MEDICAMENTS**

FSH-ZUSAMMENSETZUNGEN MIT HOHEM SIALYLIERUNGSGRAD UND DEREN VERWENDUNG ZUR HERSTELLUNG VON ARZNEIMITTELN

GONADOTROPHINES POUR LA FOLLICULOGENESE

(84) Designated Contracting States:
**AT BE BG CH CY CZ DE DK EE ES FI FR GB GR IE IT LI LU MC NL PT SE SK TR**
Designated Extension States:
**AL LT LV MK RO SI**

(30) Priority: **22.10.2001 US 338088 P**

(43) Date of publication of application:
**21.07.2004 Bulletin 2004/30**

(60) Divisional application:
**05107252.8 / 1 621 549**

(73) Proprietor: **Applied Research Systems ARS Holding N.V.
Curacao (AN)**

(72) Inventors:
• **LOUMAYE, Ernest**
**F-74140 Massongy (FR)**
• **GIARTOSIO, Carlo Emanuele**
**I-00196 Roma (IT)**

(74) Representative: **Serono International S.A.
Intellectual Property,
12, Chemin des Aulx
1228 Plan-les-Ouates (CH)**

(56) References cited:

• **DATABASE CA [Online] CHEMICAL ABSTRACTS SERVICE, COLUMBUS, OHIO, US; STANTON, P. G. ET AL: "Structural and functional characterization of hFSH and hLH isoforms"** retrieved from STN Database accession no. 126: 127295 CA XP002244324 & MOLECULAR AND CELLULAR ENDOCRINOLOGY (1996), 125(1,2, GONADOTROPINS AND THEIR RECEPTORS: STRUCTURE, FUNCTION AND MOLECULAR FORMS), 133-141 , 1996,
• **DATABASE CA [Online] CHEMICAL ABSTRACTS SERVICE, COLUMBUS, OHIO, US; ZAMBRANO, E. ET AL: "Studies on the relative in vitro biological potency of the naturally-occurring isoforms of intrapituitary follicle stimulating hormone"** retrieved from STN Database accession no. 125:317997 CA XP002244325 & MOLECULAR HUMAN REPRODUCTION (1996), 2 (8), 563-571 , 1996, cited in the application
• **DATABASE CA [Online] CHEMICAL ABSTRACTS SERVICE, COLUMBUS, OHIO, US; TIMOSSI, C. M. ET AL: "Differential effects of the charge variants of human follicle-stimulating hormone"** retrieved from STN Database accession no. 133:99824 CA XP002244326 & JOURNAL OF ENDOCRINOLOGY ( 2000 ), 165(2), 193-205 , 2000,

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 1 438 336 B1

- **DATABASE CA [Online] CHEMICAL ABSTRACTS SERVICE, COLUMBUS, OHIO, US; ZAMBRANO, ELENA ET AL: "Receptor binding activity and in vitro biological activity of the human FSH charge isoforms as disclosed by heterologous and homologous assay systems implications for the structure-function relationship of the FSH variants" retrieved from STN Database accession no. 131:165422 CA XP002244327 & ENDOCRINE (1999), 10(2), 113-121 , 1999, cited in the application**

**Description**

**Field of Invention**

The invention relates to the field of gonadotrophins, and particularly their use in assisted reproductive technologies (ART), ovulation induction (01), intrauterine insemination (IUI) and infertile male patients.

**Background of the Invention**

The gonadotrophins are a group of heterodimeric glycoproteins including follicle stimulating hormone (FSH), luteinising hormone (LH) and chorionic gonadotrophin (CG). These hormones regulate gonadal function in the male and female.

Each of these hormones is composed of two non-covalently linked subunits: an $\alpha$-subunit, which is common to FSH, LH and hCG, and a $\beta$-subunit, which is unique to each of them, and which confers biological specificity to each hormone.

In all of the gonadotrophins, each sub-unit has asparagine-linked (N-linked) oligosaccharide side chains. In the common $\alpha$-subunit of the human hormones, these are attached at positions 52 and 78. In both human FSH and CG, two N-linked oligosaccharide side chains are attached to the $\beta$-subunit, at positions 7 and 24 in FSH, and positions 13 and 30 in hCG. In human LH, one oligosaccharide is attached at position 30 of the $\beta$-subunit. hCG has additionally four serine-linked (O-linked) oligosaccharide side chains, present in the carboxyl terminal portion (CTP).

As with all glycoproteins, variations in oligosaccharide structure occur in the gonadotrophins, resulting in an array of isoforms that are found within the pituitary gland and in circulation. Furthermore, there are differences in degree of terminal carbohydrate "capping" by sialic acid. The isoforms may be separated on the basis of their charge, which is largely determined by the number and distribution of sialylated N-linked oligosaccharides. Highly sialylated forms will have a more acidic than average pl, and are termed "acidic". Less sialylated forms have comparatively higher pi's and are termed "basic".

As a consequence of their structural differences, gonadotrophin isoforms differ in their capability to bind to target-cell receptors. Degree of sialylation affects their ability to survive in circulation. In the case of FSH, several groups have demonstrated that highly acidic/sialylated isoforms have considerably longer plasma half-lives in animal models, such as the mouse and rat[1].

The isoform profile of endogenous FSH in humans has been shown to vary. Acidic isoforms with long *in vivo* half-lives and relatively low *in vitro* biological potency are predominant in the serum of prepubertal children, hypogonadal patients and in women during the follicular phase. In contrast, the less sialylated, more basic isoforms, with short *in vivo* half-lives and relatively high *in vitro* biological activity are found during puberty, GnRH therapy and around the mid-cycle gonadotrophin surge in women[2].

The FSH isoforms possessing a greater sialic acid content circulate for longer periods of time, because terminal sialic acid residues "cap" galactose residues, thus preventing an interaction with hepatic asialo-glycoprotein receptors and removal from circulation [3]

Oligosaccharide (glycan) moieties attached to proteins are branched, and each terminal sugar residue is referred to as an antenna. The parameter Z-number provides a measure of what proportion of the antennae of the carbohydrate moieties in a glycoprotein bear charged residues, such as sialic acid. Desialylated FSH has a Z-number of 0. Fully sialylated FSH would have a Z-number between about 230 to 280.

The potency of FSH preparations is estimated *in vitro* in the Steelman-Pohley assay, which compares, under specified conditions, the ability of a preparation to increase ovarian mass of immature rats in comparison with an international standard/reference preparation, calibrated in International Units (IU)[4].

Many groups have investigated the role of glycosylation and sialylation in influencing the biological profile of FSH.

D'Antonio *et al.* evaluated the metabolic clearance rates (MCR) in female rats of acidic (pl < 4.8) and basic (pl > 4.8) rhFSH isoforms obtained by chromatofocussing. As expected, the basic isoforms were found to have a faster clearance than the acidic isoforms ($t_{1/2}$ = 0.4 h for basic, 0.9 h for acidic). When the acidic and basic forms were compared (on a mass basis) in the Steelman-Pohley assay, the basic isoform was found to be considerably less active than the acidic isoform ($ED_{50}$ = 0.9. $\mu$g/rat for the basic, 0.3 $\mu$g/rat for the acidic). When the isoforms were compared on an IU basis, there was no difference between the two[5].

Vitt *et al.* carried out an *in vitro* study in which four recombinant human FSH preparations of differing pl's were compared for their ability to cause increase in size and oestradiol ($E_2$) production in isolated mouse follicles. Basic FSH (pl 5.0-5.6) was found to lead to a faster growth of follicles, and to result in the largest maximum follicle size, followed by unfractionated recombinant FSH. Mid (pl 4.5-5.0) and acid (pl 3.6-4.6) FSH preparations were behind in both growth rate and maximum size of follicles. Basic FSH was shown to induce $E_2$ secretion earlier and at a lower dose than the other isoforms. Follicles cultured with acidic FSH, regardless of concentration, secreted measurable concentrations of

$E_2$ only after prolonged incubation[6].

Timossi *et al* used chromatofocussing to separate human pituitary FSH into seven different fractions, of varying glycosylation/acidity. The fractions were tested for their ability to cause upregulation of expression of aromatase (necessary for production of oestradiol), and tissue-type plasminogen activator (tPA) *in vitro* in rat granulosa cells. The ratio of bioactivity to immunoreactivity (B/I) was found to decrease as the elution pH value of the isoform declined. The authors concluded that basic isoforms exhibited a greater capability to induce expression of both aromatase and tPA mRNA's and proteins than the acidic variants .[7]

Zambrano *et al.* fractionated human pituitary FSH into 9 fractions of varying pl, using chromatofocussing, and tested the acidic and basic isoforms using three immunoassays and two *in vitro* assays: oestradiol production by rat granulosa cells, and cAMP production by a human foetal cell line expressing the FSH receptor. The ratio of activity in the bioassays to immunoreactivity (B/I) decreased as the pi of the isoform decreased, for all bioassays[8].

In a further study, Zambrano *et al.* compared the binding affinity of seven different fractions of acidic and basic isoforms of human pituitary FSH for a heterologous receptor system (rat granulosa cells) and a homologous receptor system (recombinant human HEK-293 cells expressing the human FSH receptor). The heterologous receptor showed an increase in binding affinity as pl of the isoform increased, whereas the homologous receptor did not. cAMP production in HEK-293 cells also increased as pl of the isoform increased.[9].

Studies have shown that the more acidic forms of FSH exhibit the highest *in vivo* bioactivity (on a mass basis) when assessed by the classical ovarian weight augmentation tests.[10,11] Timossi *et al.* postulated that the basic forms may be more active *in vivo* but that because of the shorter half-life, the effect cannot be observed in weight gain of rat ovaries. They examined the effect of two preparations on a fast-response system: upregulation of tPA activity[12]. The authors concluded that rhFSH having a less acidic charge distribution profile, exhibits higher *in vitro* bioactivity and plasma clearance rates and induces tPA enzyme activity more rapidly than a highly acidic FSH preparation.

The gonadotrophins play crucial roles in the reproductive cycle, and their use is essential for assisted reproductive techniques (ART), such as *in vitro* fertilisation (IVF), IVF in conjunction with intracytoplasmic sperm injection (IVF/ICSI) and embryo transfer (ET), as well as for ovulation induction (OI) in anovulatory patients undergoing *in vivo* fertilisation either naturally or through intrauterine insemination (IUI).

ART is typically carried out using controlled ovarian hyperstimulation (COH) to increase the number of female gametes[13]. Standard regimens[14] for COH include a down-regulation phase in which endogenous gonadotrophins are suppressed by administration of a gonadotrophin releasing hormone (GnRH) agonist followed by a stimulatory phase in which follicular development (folliculogenesis) is induced by daily administration of FSH, usually at about 150-225 IU/day. Alternatively stimulation is started after spontaneous or induced menstruation while preventing the occurrence of an ill-timed LH surge by administration of a GnRH-antagonist (typically starting around day six of the stimulatory phase). When there are at least 3 follicles >16 mm (one of 18 mm), a single bolus of hCG (5-10,000 IU) is given to mimic the natural LH surge and induce ovulation. Oocyte recovery is timed for 36-38 hours after the hCG injection.

01 is typically carried out with daily administration of FSH at a dose of about 75-150 IU/day. Down-regulation with GnRH agonists or antagonists may be used, although less frequently than in the ART indication. hCG is given to mimic the LH surge prior to *in vivo* fertilisation, which is achieved either through regular intercourse or IUI.

The typical regimens described above for ART and OI require daily injections of gonadotrophins over a prolonged period, i.e. for an average of 10 days, and up to 21 days in some patients. The development of FSH preparations of increased efficacy would permit the daily dosage of FSH to be decreased, and/or permit a shortening of the treatment period (i.e. fewer injections), and/or allow injections to be given less frequently. This would render ART and OI regimens more convenient and patient-friendly.

Furthermore, ART using *in vitro* fertilisation is fraught with possible mishaps. For example, not every follicle will produce a viable oocyte, not every viable oocyte will be successfully fertilised, and some embryos may not be viable. Moreover, once viable embryos are selected, transfer to the uterus and implantation may not be successful. In order to maximise the chances of a live birth it is therefore desirable to stimulate the growth and maturation of several follicles, to ensure the collection of multiple oocytes.

In the indication of OI, in contrast, the objective is to obtain not more than three and preferably one dominant follicle (to avoid multiple pregnancies).

Some patients undergoing ART and OI present a reduced number of growing follicles when treated with conventional FSH preparations. This is a limiting factor for success when undergoing ART, in that it limits the number of embryos available for transfer and/or cryopreservation. It can also be a limiting factor for success in patients undergoing IUI, where obtaining more than one follicle is important. Patients presenting this type of response include patients above about 33-35 years old, patients with elevated base-line FSH, elevated base-line oestradiol or reduced base-line *inhibin b.*

In the male, spermatogenesis is dependent on stimulation of Sertoli cells by FSH. FSH deficiency results in oligospermia, and hence infertility. The treatment of male infertility with conventional FSH preparations requires FSH injections three times a week for up to 18 months.

The development of FSH preparations with enhanced ability to stimulate folliculogenesis, is an ongoing need.

There is also an existing need for new FSH preparations to treat patients with a diminished response to FSH. Also desirable are FSH preparations of enhanced efficacy, permitting shorter treatment protocols and/or decreased cumulative doses and/or less frequent dosing, for ART, OI and male infertility.

**Summary of the invention**

It is an object of the invention to provide a gonadotrophin preparation for use in ovulation induction and COH, particularly in conjunction with ART.

In a first aspect, the invention provides a recombinant FSH preparation, wherein the Z-number of the preparation is at least at 200.

In a second aspect, the invention provides a recombinant FSH preparation, wherein the preparation has an average pl below 3.4.

In a third aspect, the invention provides a pharmaceutical composition comprising recombinant FSH, wherein the recombinant FSH has a Z-number that is at least at 200.

In a fourth aspect, the invention provides a use of recombinant FSH in stimulation of folliculogenesis, wherein the recombinant FSH has a Z-number that is at least at 200.

In a fifth aspect, the invention provides a use of recombinant FSH in the preparation of a medicament for use in stimulation of folliculogenesis, wherein the recombinant FSH has a Z-number that is at least at 200.

in a sixth aspect, the invention provides a method for inducing folliculogenesis in a human patient, the method comprising administering recombinant FSH to the patient, wherein the recombinant FSH has a Z-number that is at least at 200.

In a seventh aspect, the invention provides a method for preparing a recombinant FSH preparation having a Z-number that is at least at 200, the method comprising a step selected from:

- reacting FSH with a sialic acid donor in the presence of 2,3-sialyltransferase;
- selecting a suitable cell type for expression of recombinant FSH;
- culturing a cell, preferably recombinant, that is expressing FSH under conditions that favour high levels of sialylation; and
- isolation of FSH isoforms having a high Z-number using a chromatographic technique.

In an eighth aspect, the invention provides a use of recombinant FSH in treating male infertility, wherein the recombinant FSH has a Z-number that is at least at 200.

In a ninth aspect, the invention provides a use of recombinant FSH in the preparation of a medicament for use in the treatment of male infertility, wherein the recombinant FSH has a Z-number that is at least at 200.

In a tenth aspect, the invention provides a method for treating male infertility in a human patient, the method comprising administering recombinant FSH to the patient, wherein the recombinant FSH has a Z-number that is at least at 200.

**Brief description of the drawings**

Figure 1 shows a chromatogram for elution through a GlycoSep® C column of glycans released from rFSH; column 4.6 x 100 mm, packed with polymer coated divinyl benzene resin (5 m), with a mobile phase of acetonitrile:water 20:80, with a linear gradient of 0.25% per minute of ammonium acetate (500 mM) from 5 to 21 minutes, followed by a linear gradient of 0.525% per minute of ammonium acetate (500 mM) from 21 to 61 minutes. The X-axis shows retention time in minutes, and the Y-axis shows signal strength in mV.

Figure 2 shows the number of follicles per size category, on day 8 (on the Y-axis), in patients receiving acidic and basic isoforms of FSH until day 7. Wavy lines represent the result with acidic isoforms, oblique lines represent the result with basic isoforms.

Figure 3 shows the number of follicles per size category, on day 10 (on the Y-axis), in patients receiving acidic and basic isoforms of FSH until day 7. Wavy lines represent the result with acidic isoforms, oblique lines represent the result with basic isoforms.

Figure 4 shows average FSH serum levels in patients after the last dose of acidic and basic isoforms of FSH. The X-axis represents time in hours since first FSH injection, the Y axis represents serum concentration in immuno-reactive IU/L. Squares (■) show serum concentration after injection of acidic isoforms; diamonds (♦) show serum concentration after injection of basic FSH isoforms. Serum concentrations were measured by immunoassay, for example, radio-immunoassay, using a kit as supplied by Daiichi Isotope Laboratory, Japan.

Figure 5 shows the amino acid sequence for the mature human FSH alpha subunit.
Figure 6 shows the amino acid sequence for the mature human FSH beta subunit.

**Detailed description of the invention**

The inventors have surprisingly found that highly sialylated recombinant FSH isoforms have a greater efficacy in inducing folliculogenesis in human patients than less sialylated isoforms. The use of an FSH preparation of the invention permits the use of lower cumulative doses of FSH to achieve the same or better clinical result.

The inventors have found that when patients are treated with equal amounts of acidic FSH and basic FSH, on an IU basis, as determined by the conventional assay, the number of growing follicles in patients treated with acidic FSH is significantly greater.

When patients are treated with equal amounts of acidic FSH and basic FSH, on a mass basis, the number of follicles in patients treated with acidic FSH is also substantially greater.

Some patients present a reduced number of growing follicles when treated with conventional FSH preparations. This is a limiting factor for success when undergoing ART. Patients presenting this type of response include patients above about 33-35 years old, patients with elevated base-line FSH, elevated base-line oestradiol or reduced base-line *inhibin b.* The preparation of recombinant FSH according to the invention may be injected once daily, or every other day to elicit a better ovarian response than with conventional preparations. This increases the chances of conception for these patients.

The inventors have also surprisingly found that recombinant FSH preparations having greater efficacy, allowing less frequent dosing, can be made by using recombinant FSH having a Z-number that is at least at 200, preferably at least at 210, 220, 230, 240, 250, 260, 270, 280 and 290 with the order of preference increasing with increasing Z-number (Z-numbers falling between these values are of course within the scope of the invention). For Inducing folliculogenesis, conventional FSH preparations are generally administered every day, at a dosage of about 75-600 IU/day. In the majority of patients, the same cumulative dose of a conventional FSH preparation may be administered every two days, while achieving a similar clinical result as daily injections[15]. The expression "less frequent dosing" is meant to apply to FSH preparations that may be administered less frequently than every two days, while achieving the same clinical result, in terms of total follicular volume, as conventional preparations administered every one or two days.

The terms "acidic" and "basic" are widely used to refer to FSH preparations having varying degrees of sialylation. Because sialic acid is acidic, more highly sialylated molecules will have lower pl's. Using isoelectric focussing, chromatofocussing or other separating methods, such as ion-exchange chromatography, FPLC and HPLC[16], a mixture of isoforms may be separated into fractions which may be assigned as acidic or basic, preferably based on Z-number.

The term "sialic acid" refers to any member of a family of nine-carbon carboxylated sugars. The most common member of the sialic acid family is N-acetylneuraminic acid (2-keto-5-acetamido-3,5-dideoxy-D-glycero-D-gatactononulopyranos-1-onic add, often abbreviated as Neu5Ac, NeuAc, or NANA). A second member of the family is N-glycolyl-neuraminic acid (Neu5Gc or NeuGc), in which the N-acetyl group of NeuAc is hydroxylated. A third sialic acid family member is 2-keto-3-deoxy-nonulosonic acid (KDN).[17] Also included are 9-substituted sialic acids such as a 9-O-$C_1$-$C_6$-acyl-Neu5Ac like 9-O-lactyl-Neu5Ac or 9-O-acetyl-Neu5Ac, 9-deoxy-9-fluoro-Neu5Ac and 9-azido-9-deoxy-Neu5Ac. For review of the sialic acid family, see, e.g., Varki; *Glycobiology* **2** *1992*; 25-40; Sialic Acids: Chemistry, Metabolism and Function, R. Schauer, Ed. (Springer-Verlag, New York (1992)).

Carbohydrate (alternatively "glycan") moieties are attached to the peptide backbone via a single sugar, either with an O- or N-linked glycosidic bond. As the carbohydrate moiety becomes elaborated, branching may occur, with the result that the carbohydrate moiety has one, two, three, or four (sometimes more) terminal sugar residues or "antennae". Such carbohydrate moieties are referred to as mono- di- tri- or tetra-branched. The parameter antennarity index (AI) provides a measure of the degree of branching in carbohydrate residues, giving also a measure of the 3-D size of the carbohydrate moieties. To determine this parameter, a glycoprotein is treated chemically to release all carbohydrate residues, for example by heating with hydrazine, or the carbohydrate may be cleaved enzymatically, for example, with endoglycosidase (N-glycanase)[18]. The carbohydrate mixture is isolated. If desired, the carbohydrate mixture is reacted with a label, such as a radio-label, a chromophore-label (i.e. UV-vis active), a fluorophore label, an immunoreactive label, etc. The labelled carbohydrate mixture is then desialylated, with the enzyme sialidase, to yield a labelled neutral carbohydrate mixture (Alternatively, the steps of labelling and desialylation may be reversed in order). The labelled neutral carbohydrate mixture is then separated into its components, using a chromatographic method that can distinguish between the different species (mono-, di-, tri- and tetra-branched). Chromatography (normal- or reverse-phase) may be performed using essentially any method, including, for example thick or thin layer chromatography, or high performance liquid chromatography (HPLC). Alternatively, the isolated neutral carbohydrate mixture may be reacted with an agent to render the components volatile, and the mixture may be subjected to gas chromatography (GC). Visualisation will be accomplished with a method appropriate to the label and the chromatographic method used. For example, if a fluorophore is used as label, a fluorimeter will be used for detection; if a chromophore is used as label, a UV-vis spectrophotometer will be used for detection. If no label is used, mass spectrometry may be used to measure peaks and retention times. Peak assignments to mono- di- tri- or tetra-branched species can be done using mass spectrometry, or by comparing with known standards.

A chromatogram is then analysed by integrating the peaks associated with di- tri-and tetra-branched carbohydrate species. The percentage of the total carbohydrate represented by each species can then be used to calculate AI according to the following equation:

$$AI = 2\,P_{di} + 3\,P_{tri} + 4\,P_{tetra}$$

wherein AI is antennarity index, and $P_{di}$, $P_{tri}$ and $P_{tetra}$ are the percentage of total carbohydrate that is di-, tri- and tetra-branched respectively. Trace amounts of other components (e.g. mono-antennary) may be present but do not contribute significantly to the AI value.

A high antennarity index indicates that the carbohydrate moieties are highly branched, with many antennae. Recombinant human FSH typically has an AI of from about 220-280, or on average about 255.

The parameter Z-number provides a measure of how many of the antennae of the carbohydrate moieties in a glycoprotein bear charged residues, such as sialic acid. To determine Z-number, the carbohydrate moieties are released from the peptide, as above, and labelled, if desired. The mixture is then separated by ion exchange chromatography, allowing the separation of species on the basis of charge. Visualisation of the eluted peaks may be by virtue of a label, as mentioned above, or may be by some other method, such as mass-spectrometry. A chromatogram is then analysed by integrating the peaks associated with mono- di- tri- and tetra-charged carbohydrate species. The percentage of the total carbohydrate represented by each species can then be used to calculate Z-number according to the following equation:

$$Z = P'_{mono} + 2\,P'_{di} + 3\,P'_{tri} + 4\,P'_{tetra}$$

wherein Z is Z-number, and $P'_{mono}$, $P'_{di}$, $P'_{tri}$ and $P'_{tetra}$ are the percentage of total carbohydrate that is mono-, di-, tri- and tetra-charged respectively.

A high Z-number indicates that a large number of antennae bear charged residues, and that the glycoprotein will therefore be highly charged, and in the case of sialic acid residues, acidic. Recombinant human FSH typically has Z-number values in the range of about 150 to about 190, or on average about 184.

The inventors have surprisingly found that recombinant FSH isoforms that have Z-numbers higher than 200 show increased efficacy in terms of number of follicles, when compared on an IU basis with an "equivalent dose" of FSH isoforms having Z-numbers less than 200. By "equivalent dose" is meant that when the FSH amount of different isoforms is measured by the conventional *in vivo* assay by comparing, under specified conditions, their ability to increase ovarian mass in rats, the IU dose is the same. In other words, equivalent IU doses of different isoforms, as determined in rats, have different clinical efficacy, when administered to humans.

FSH preparations having a increased Z-numbers may be isolated by any number of ways. For example, a batch of recombinant FSH may be subjected to isoelectric focussing, or chromatofocussing as described, for example, by any one of Mulders *et al.*[19], Zambrano *et al.*[20], and Timossi *et al.*[21] Fractions having various pi's may be isolated. Preferred recombinant FSH preparations of the invention have average pl's of less than 3.4, more preferably less than 3.3, particularly preferably less than 3.2 with degree of preference increasing as the average pl decreases.

The parameter Z-number reflects the average degree of sialylation of a population of FSH species. It is possible that an FSH preparation having a high Z-number may still have a substantial proportion of basic (less sialylated) species. Such basic species may act as antagonists at the FSH receptor, and are therefore undesirable. The "spread" of species present may be determined by isoelectric focussing, or chromatofocussing. The Z-number analysis can also give an idea of the spread of species. It is preferred that the preparation have less than at or about 4% neutral carbohydrate species (i.e. glycan moieties bearing no charge), and less than at or about 16% mono-sialylated species, and more preferred that the preparation have less than at or about 3%, 2% or 1% neutral species and less than at or about 15%, 12%, 10%, 8% or 5% monosialylated species, with degree of preference increasing with decreasing percentages.

Within the scope of the invention are recombinant FSH preparations having increased efficacy in folliculogenesis resulting from an increased degree of sialylation at one or more additional glycosylation sites on the protein. Such sites may be introduced by substitution of residues in the FSH protein backbone with serine, threonine, lysine or asparagine residues, using, for example, mutagenesis. An example of a method that may be used to generate such mutant forms of FSH is given in Example 7. For *in vivo* glycosylation, the site introduced should be such as to form an "*N*-glycosylation site", of the following sequence: N-X'-S/TVC-X", wherein X' is any amino acid residue except protine, X" is any amino acid residue which may or may not be identical to X' and which preferably is different from proline, N is asparagine, and S/T/C represents a residue that may be serine, threonine or cysteine, preferably serine or threonine, and most preferably threonine. Acidic isoforms (pI ≤ 3.4) of such FSH molecules fall within the scope of the invention. Such modified FSH molecules, bearing additional glycosylation sites are described, for example in WO 01/58493 (Maxygen). Particularly preferred are the following mutations:

In the β-subunit: E4N, A70N, L73N, V78N, G100N, Y103N, F19N/l21T, L37N/Y39T, D41N/A43T, E55N/A43T, E59NN61T and R97N/L99T;
In the α-subunit: E9N, F17T, F17N, R67N, V68T, E56N, H83N, and F33N/R35T;

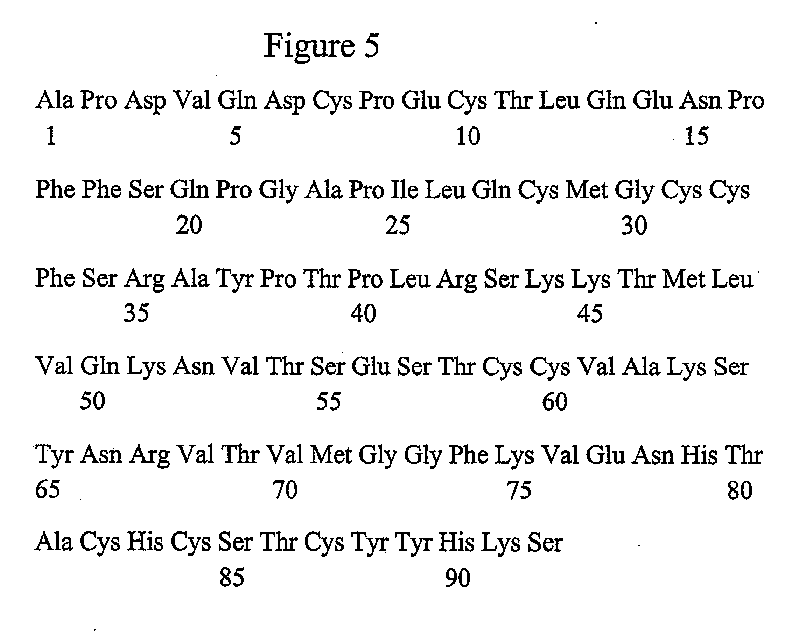

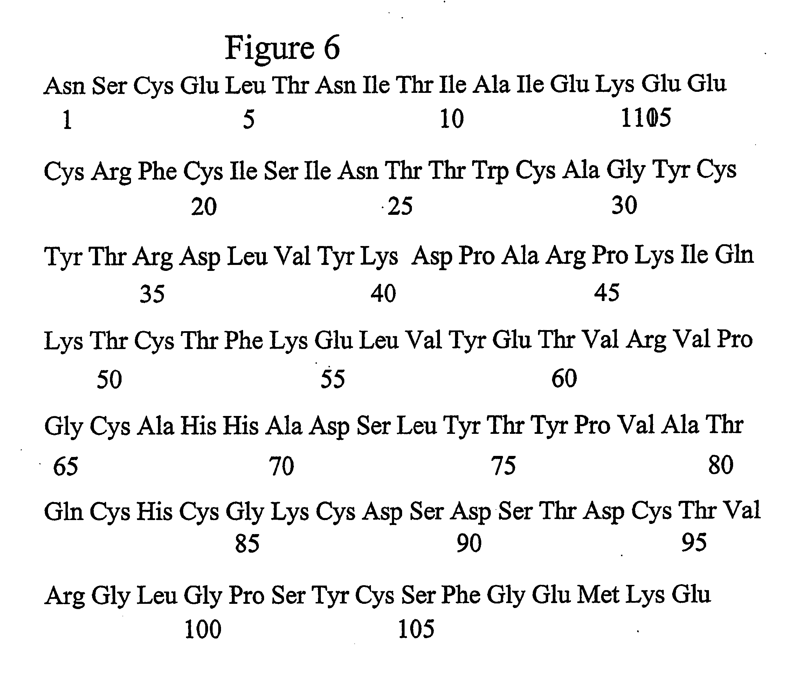

wherein A is alanine, D is aspartic acid, E is glutamic acid, F is phenylalanine, G is glycine, H is histidine, I is isoleucine, L is leucine, N is asparagine, R is arginine, T is threonine, V is valine, Y is tyrosine, and the notation "E4N" represents a replacement of a glutamic acid (E) at position 4 with an asparagine (N). For sequence numbering, the amino acid sequence of human FSH alpha is numbered according to the mature sequence shown in Figure 5 or SEQ ID NO:1. The amino acid sequence of human FSH beta is numbered according to the mature sequence shown in Figure 6 or SEQ ID NO: 2.

Also within the scope of the invention are recombinant FSH preparations having increased efficacy in folliculogenesis resulting from an increased degree of sialylation atone or more additional glycosylation sites present on an appended peptide. By "appended peptide" is meant any peptide which includes a glycosylation site, and which may be attached to the amino and/or carboxyl terminus of the α- and/or β-subunit of FSH without deleteriously affecting the FSH activity of the resulting molecule. For example, the β-subunit of hCG is substantially larger than that of the other gonadotrophins, due to approximately 34 additional amino acids at the C-terminus referred to herein as the carboxyl terminal portion (CTP). In urinary hCG, the CTP contains four mucin-like O-linked oligosaccharides. This CTP may be ligated to the β-subunit of FSH, preferably at the carboxyl terminal of the β-subunit of FSH, resulting in a molecule having FSH activity and having an additional four sites of glycosylation. Acidic isoforms (pI ≤ 4.4) of such FSH molecules fall within the scope of the invention. Such molecules are disclosed in WO 93/06844 (Washington University), and by Boime et al.[22] Other FSH molecules having modified glycosylation sites are disclosed in WO 90/09800 (Washington University).

For the purposes of this description, FSH preparations having additional glycosylation sites will be denoted FSH$^{gly+}$. When additional glycosylation sites are added, the parameter Z-number can no longer be used to compare with "normal" FSH preparations (i.e. those having four glycosylation sites), as this parameter is normalised (it is a sum of percentages). When an FSH$^{gly+}$ preparation is subjected to a glycan species analysis, the parameter Z$^+$-number may be calculated, in a manner analogous to Z-number. The FSH$^{gly+}$ preparations of the invention have Z$^+$-numbers of more than 200, preferably more than 210, 220, 230, 240, 250, 260, 270, with degree of preference increasing as Z$^+$-number increases.

FSH$^{gly+}$ preparations of the invention have significantly lower pi profiles than normal FSH. Particularly preferred for their increased efficacy are those FSH$^{gly+}$ preparations having average pI's of less than 4.4, more preferred are those having average pi's less than 4.2, 4.0, 3.8, 3.6, 3.4, 3.3 and 3.2, with degree of preference increasing with decreasing average pl.

In all embodiments of the invention recombinant FSH is used. For treating human patients, human recombinant FSH is preferred. Preparations of the invention may be isolated from conventional recombinant FSH, or they may be isolated from FSH$^{gly+}$ preparations.

It is also an aspect of the invention to provide a method for enriching sialic acid content using a method that the inventors call "sialyl boosting". Recombinant FSH or recombinant FSH$^{gly+}$ preparations may be subjected to sialyl boosting, by treating with an enzyme, such as a glycosyl transferase, in particular sialyltransferase, in the presence of a sialic acid donor, for example CMP-sialic acid, as described in WO 98/31826 (Cytel Corporation). Examples of recombinant sialyltransferases, as well as methods of producing recombinant sialyltransferases, are found in, for example, US Patent No. 5,541,083 (University of California; Amgen). At least 15 different mammalian sialyltransferases have been documented, and the cDNAs of thirteen of these have been cloned. These cDNAs can be used for recombinant production of sialyltransferases, which can then be used in the methods of the invention.

The sialyl transferase that is used will be able to transfer sialic acid to the sequence Galβ1, 4GlcNAc, which are the most common penultimate moieties underlying the terminal sialic acid on sialylated glycoproteins. An example of a sialyltransferase that may be used is ST3Gal III, which is also referred to as a (2,3)-sialyltransferase (EC 2.4.99.6). This enzyme catalyses the transfer of sialic acid to the Gal of a Gal-β-1,3-glycosylNAc or Gal-β-1,4-glycosylNAc glycoside.[23] The sialic acid is linked to a galactosyl (Gal) residue with the formation of an α-linkage between the two saccharides. Linkage of the saccharides is between the 2-position of NeuAc and the 3-position of Gal. This particular enzyme can be isolated from rat liver[24]; the human cDNA[25] and genomic[26] DNA sequences are known, facilitating production of this enzyme by recombinant expression. In a preferred embodiment, the sialylation methods use a ST3Gal III (preferably from rat), ST3Gal IV, ST3Gal I, ST6Gal I, ST3Gal V, ST6Gal II, ST6GalNAc I, or ST6GalNAc II, more preferably ST3Gal III, ST6Gal I, ST3Gal IV, ST6Gal II or ST3Gal V, more particularly preferably ST3Gal III from rat.

The amount of sialyl transferase will preferably be in the range of at or about 50 mU per mg of FSH or less, preferably at or about 5-25 mU per mg of FSH. Under preferred conditions the concentration of sialyl transferase will be at or about 10-50 mU/ml, and the FSH concentration will be at or about 2 mg/ml.

It is also possible to produce FSH enriched in acidic isoforms by transfecting a cell, recombinant or otherwise, that is expressing FSH, with a gene encoding a sialyltransferase, which gene is expressible in the cell. The gene may comprise genomic coding sequences (i.e. with introns) or it may comprise cDNA coding sequences. Alternatively, if the genome of the cell contains endogenous sequences encoding sialyltransferase, a construct causing the expression of FSH may be inserted into the genome of the cell. The expression of sialyltransferase may be increased by inserting non-native regulatory sequences, active in the cell, in operative connection to the endogenous sequences encoding sialyltransferase. It is also possible to insert an amplifiable gene in operative connection to the sequences encoding sialyltransferase, so as to cause amplification of the genomic sialyltransferase encoding sequences. These manipulations may be performed using homologous recombination, for example, as described in EP 0 505 500 (Applied Research Systems ARS Holding N.V.).

The degree of sialylation of an FSH preparation may also be increased by selecting a cell for expression of recombinant FSH that is known to favour sialylation. Such cells include selected pituitary cells and Chinese Hamster Ovary cells that express high levels of sialyltransferases. An FSH preparation prepared in such a cell may further be subjected to an isolation method, as mentioned herein, in order to isolate isoforms having a high degree of sialylation.

The degree of sialylation of an FSH preparation may also be increased by culturing a cell that is expressing FSH, preferably recombinant FSH, under conditions that favour a high level of sialylation. Sialylation may be favoured by supplementing the culture media with inhibitors of neuraminidase and/or direct intracellular precursors for sialic acid synthesis, such as acetylmannosamine. An FSH preparation prepared under such culturing conditions may further be subjected to an isolation method, as mentioned herein, in order to isolate isoforms having a high degree of sialylation.

If sialyl boosting is used, it is desirable that prior to enzymatic sialylation, the FSH preparation have a high AI, so as to provide many antennae for attachment of sialic acid residues. Preferably the FSH should have an AI of more than 220, more preferably it should have an AI of more than 240, and more particularly preferably it should have an AI of more than 270. FSH having higher AI's may be isolated, for example, using affinity chromatography on concanavalin-A (Con-A) derivatised Sepharose, eluting with a gradient of methyl-glucose, or by preparative HPLC.

The sialylation boosting method of the invention may advantageously be applied to FSH preparations that have been modified to introduce one or more additional sites of glycosylation (FSH$^{gly+}$ preparations). Such FSH$^{gly+}$ preparations may also be separated into those fractions having high AI's prior to sialyl boosting.

The invention includes recombinant FSH preparation made by expressing FSH in cells that are not capable of sialylation, and then subjecting the FSH to sialyl boosting. For example, WO 99/13081 (Akzo Nobel N.V.) describes the expression of wild type FSH and muteins in the uni-cellular eukaryote *Dictyostelium,* particularly muteins having additional glycosylation sites. *Dictyiostelium* is not capable of sialylating glycans. The invention includes recombinant FSH preparations made by subjecting wild type FSH or muteins expressed in *Dictyostelium* to sialyl boosting.

After sialyl boosting recombinant, FSH preparations having the desired degree of sialylation may be isolated using ion exchange chromatography, isoelectric focussing, chromatofocussing, or chromatography on Concanavalin-A (Con-A).

The recombinant FSH of the Invention has a Z-number of at least 200, more preferably at least 210, particularly preferably at least 220, more particularly preferably at least 230, 240, 250, 260 or 270, with degree of preference increasing with increasing Z-number. Fully sialylated FSH has a Z-number of 230 to 280, depending on the antennarity index. Very preferred FSH preparations according to the invention have a Z-number of 230 to 280.

The recombinant FSH preparations of the invention are prepared to consistently have a Z-number of at least 200, or the preferred Z-numbers mentioned above. The FSH of the invention may be isolated from a mixture of isoforms using a number of methods that will be known to one skilled in the art. For example, isoelectric focussing, chromatofocussing or ion-exchange chromatography may be used to separate the isoforms on the basis of pI. The different fractions can be analysed for sialic acid content, and the desired fractions selected for use. An example of suitable conditions for ion-exchange chromatography is given in the Examples, Such separation methods can be used to isolate FSH of the invention from conventionally produced rFSH, or it may be used to isolate desired isoforms from FSH treated with sialyltransferase or the other recombinant techniques mentioned above.

In one aspect, the invention provides a pharmaceutical composition comprising recombinant FSH according to the invention (i.e. having a Z-number of at least 200, preferred values for minimum Z-number are as listed above). Such pharmaceutical compositions can be used to stimulate folliculogenesis, for example in conjunction with ovulation induction or assisted reproductive techniques (ART). Because the FSH of the invention is particularly effective in inducing multiple follicles to develop and mature, it is particularly suitable for use in ART, in which it is desired to collect multiple oocytes.

Alternatively, with careful tailoring of the dose, recombinant FSH of the invention may be used to induce mono-fiolliculogenesis for OI, or paucifolliculogenesis (up to about three follicles) for IUI, for *in vivo* fertilisation. Mono-folliculogenesis can also be attained with a reduced dose of FSH, or less frequent dosing as compared with conventional FSH preparations. For example, in OI a recombinant FSH preparation of the invention may be administered at 225-400 IU every three days, or lower doses, depending on the patient response. Patient response may be followed by sonography.

The recombinant FSH of the invention will typically be formulated as a pharmaceutical composition, which will additionally comprise a diluent or excipient. A person skilled in the art is aware of a whole variety of such diluents or excipients suitable to formulate a pharmaceutical composition.

Recombinant FSH of the invention is typically formulated as a unit dosage in the form of a solid ready for dissolution to form a sterile injectable solution for intramuscular or subcutaneous use. The solid usually results from lyophilisation. Typical excipients and carriers include sucrose, lactose, sodium chloride, buffering agents like sodium phosphate monobasic and sodium phosphate dibasic. The solution may be prepared by diluting with water for injection immediately prior to use.

Recombinant FSH of the invention may also be formulated as a solution for injection, comprising any of the excipients and buffers listed above, and others known to one skilled In the art

The recombinant FSH of the invention may be used in a controlled ovarian hyperstimulation (COH) regimen. Standard regimens[27] for COH include a down-regulation phase in which endogenous luteinising hormone (LH) is down-regulated by administration of a gonadotrophin releasing hormone (GnRH) agonist followed by a stimulatory phase in which follicular development (folliculogenesis) is induced by daily administration of follicle stimulating hormone (FSH), usually at or about 75-600 IU/day, preferably at or about 150-225 IU/day. Alternatively stimulation is started with FSH after spontaneous or induced menstruation, followed by administration of a GnRH-antagonist (typically starting around day six of the stimulatory phase). When there are at least 3 follicles >16 mm (one of 18 mm), a single bolus of hCG (5-10,000 IU) is given to mimic the natural LH surge and induce ovulation. The hCG injection is typically administered on any one of days 10 to 14, but may be administered later, depending on when the above parameters are met. Oocyte recovery is timed for 36-38 hours after the hCG injection.

The recombinant FSH of the invention may also be used for OI and IUI. For example, FSH stimulation with a preparation of the invention is started after spontaneous or induced menstruation, at a daily dose of 75-150 IU. When 1 or 3 follicles have reached a diameter of at least 16 mm, a single bolus of hCG is administered to induce ovulation. Insemination is performed *in vivo,* by regular intercourse or IUI.

Because the recombinant FSH of the invention has an increased efficacy with respect to known FSH preparations, regimens such as that described above may employ lower IU doses of FSH, and/or may be modified by decreasing the FSH stimulation period, while achieving the same or better response, in terms of number and viability of follicles. For example, using an FSH preparation of the invention, adequate folliculogenesis may be achieved with at or about 50-150 IU FSH, preferably at or about 50-100, more preferably at or about 50-75 IU FSH. Dosing of FSH is usually on a daily or semi-daily basis. The dosing period may be less than at or about 14 days, preferably less than at or about 12 days, more preferable less than at or about 11 or 10 days.

For OI, the recombinant FSH preparations of the invention may be administered at doses from 25-150 IU FSH/day, preferably, 50-125 IU FSH/day.

For the treatment of male infertility, a recombinant FSH preparation of the invention may be administered at 3X 150 to 300 IU/week until spermatogenesis reaches levels adequate for insemination, either through regular intercourse or ART techniques.

The inventors have further found that because of increased efficacy, recombinant FSH preparations having a Z-number of at least 200 may be administered less frequently than FSH preparations having a Z-number of less than 200. (For the purposes of this description, the terms FSH$^{+200}$, FSH$^{+200}$, FSH$^{+220}$, etc. will be used to represent FSH preparations having Z-numbers in the range of 200-210, 211-220, 221-230, etc.) This means that patients who would normally require, for example 150 IU of conventional FSH every day to achieve adequate folliculogenesis, can achieve the same result with, for example, 225 IU of FSH$^{+200}$, every three days, or 300 IU of FSH$^{+200}$, every four days. Because of the increased efficacy of FSH$^{+200}$, as compared with conventional FSH preparations, the above-quoted dosages may be decreased In those patients showing a good response. With recombinant FSH preparations of the invention having Z-numbers not less: than 230, it may be possible to give injections only every five, six, or seven days, depending on the response of the patient. Response may be evaluated by sonography, and/or by measuring serum estradiol levels. Other suitable regimens are as follows: 100 IU FSH$^{+210}$ every two days; 200 IU FSH$^{+210}$ every three days; 275 or 300 IU FSH$^{+210}$ every four days; 80-100 IU FSH$^{+220}$ every two days; 180-200 IU FSH$^{+220}$ every three days; 260-300 IU FSH$^{+220}$ every four days; 75-100 IU FSH$^{+230}$ every two days, 170-200 IU FSH$^{+230}$ every three days; and 250-300 IU FSH$^{+230}$ every four days; 275-400 IU FSH$^{+250}$ every five days; 375-450 IU FSH$^{+250}$ every six days; 450-525 IU FSH$^{+250}$ every seven days.

The term "increased efficacy", as used herein in connection with an effect on folliculogenesis includes any measurable improvement or increase in the number and/or viability of follicles in an individual, for example, when compared with the number and/or viability of follicles in one or more patients treated with an equivalent dose (IU/IU), as determined in the conventional assay of ovarian weight increase in rats, of FSH having a Z-number of less than 200. Preferably the improvement or increase will be a statistically significant one, preferably with a probability value of <0.05. Methods of determining the statistical significance of results are well known and documented in the art and any appropriate method may be used.

The invention will be illustrated with the following, non-limiting examples.

Examples

**Example 1**

**Determination of Z-number**

Glycan mapping allows the determination of the Z-number of a glycoprotein.

Glycan moieties were released from recombinant human FSH, using Oxford GlycoSciences GlycoPrep® 1000 fully automated instrument or equivalent, with hydrazine at 100°C for 5 hours.

The glycan species were separated from unreacted hydrazine and amino acid hydrazides using a coated glass bead column. Glycan species were eluted with a sodium acetate reagent

The glycan species were acetylated with acetic anhydride. Excess reagents were removed, using a mixed-bed ion-exchange column. Any unreduced glycan species is collected in a dilute acetate buffer solution.

Glycan species were collected on a 0.5 m filter (Oxford GlycoSciences) and lyophilised. The dried glycan species were labelled by reacting with a reductant having a fluorophore (for example, 2-aminobenzamide or 2-AB) under acidic conditions, for 120 min at 65°C.

The labelled glycan species were separated from excess reagents using a hydrophilic adsorption membrane that retains the glycan species. The glycan species were recovered in water and stored frozen until chromatographic separation.

The labelled glycan species were separated by anion-exchange chromatography. The chromatographic procedure is performed as follows:

- The column is a GlycoSep® C column, 4.6 x 100 mm, packed with polymer coated divinyl benzene resin (5 m)
- The mobile phase has a flow rate of 0.4 ml/min:

    Mobile phase A: Acetonitrile (chromatographic grade)
    Mobile phase B: Ammonium acetate 500 mM, pH 4.5
    Mobile phase C: Ultrapure water

- Detection is with a fluorimeter set at $\lambda_{excitation}$: 330 nm and $\lambda_{emission}$: 420 nm;
- Elution is under the following elution conditions:

    Initial conditions: 20 % phase A, 80% phase C
    Linear gradient phase B (0.25 % per min) from 5 to 21 min, 20% phase A constant.
    Linear gradient phase B (0.525 % per min) from 21 to 61 min, 20% phase A constant.

- The column is maintained at a temperature of 30±2°C.

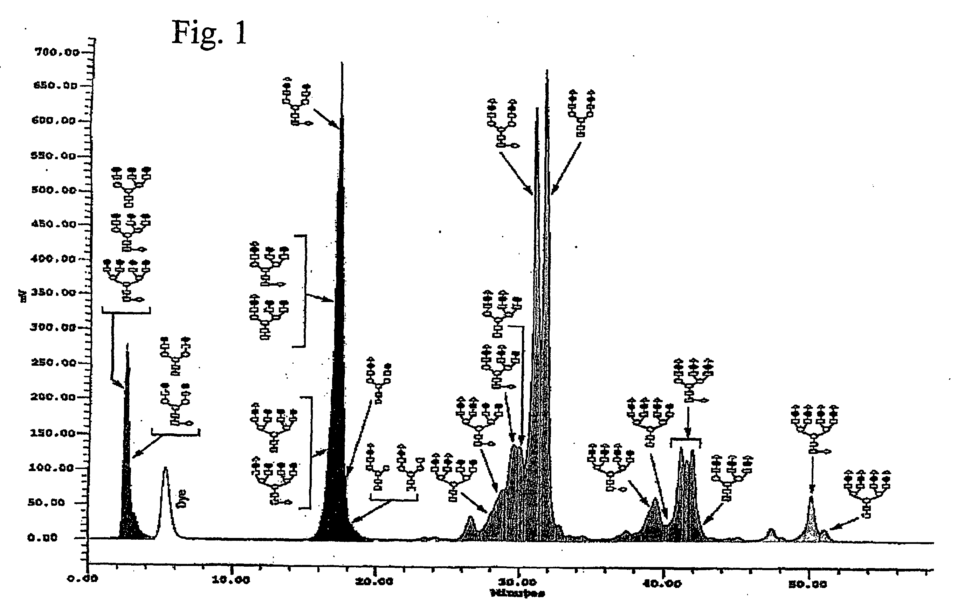

The glycan species elute according to their charge from neutral, mono-, di-, tri-and tetrasialylated species. A typical chromatogram is shown in Figure 1.

In the obtained chromatogram, the peaks were grouped according to the range of retention times which correspond to the degrees of sialylation listed in Table 1.

| Table 1: retention times and charge numbers for glycans released from rFSH | | |
|---|---|---|
| Retention times (min.) | Glycan species | Charge number |
| 2 to 4 | Neutral | 0 |
| 15 to 21 | Monosialylated | $P_{mono}$ |
| 21 to 35 | Disialylated | $P_{di}$ |

Table continued

| Table 1: retention times and charge numbers for glycans released from rFSH | | |
|---|---|---|
| Retention times (min.) | Glycan species | Charge number |
| 35 to 45 | Trisialylated | $P_{tri}$ |
| 45 to 52 | Tetrasialylated | $P_{tetra}$ |

The results for each glycan group were expressed as the percent of the total area of the different glycan groups (neutral, mono-, di-, tri- and tetra-) and a Z-number is calculated from the proportions of the different species ($P_{glycan}$):

$$Z \quad = \quad P'_{mono} \quad + \quad 2\,P'_{di} \quad + \quad 3\,P'_{tri} \quad + \quad 4\,P'_{tetra}$$

**Example 2**

**Determination of antennarity index (AI)**

The glycans were released from the peptide backbone by hydrazinolysis, and then fluorescently labelled using 2-aminobenzamide (2-AB), as detailed in Example 1.

The 2-AB labelled glycans were desialylated enzymatically with sialidase (*Vibrio cholerae*) in 250 mM ammonium acetate, pH 5.5 containing 20 mM calcium chloride for 18 hours at 37°C. Approximately 0.05 U sialidase are used for glycans from a starting quantity of 100 $\mu$g of rhFSH.

The desialylated glycans were dried under vacuum and stored at -20°C before separation by preparatory reverse-phase HPLC, under the following conditions:

- The column was a GlycoSep® R column;
- The mobile phase had a flow rate of 0.7 ml/min.

> Eluent A: ammonium acetate 50 mM, pH 6.0;
> Eluent B: ammonium acetate 50mM, pH 6.0 containing 8% acetonitrile;

- Detection was with a fluorimeter set at $\lambda_{excitation}$ = 330 nm ; $\lambda_{emission}$ = 420 nm.
- Column temperature: 30°C.

Prior to application to the column, dried samples were reconstituted with Eluent A (200 $\mu$l) : 50 $\mu$l of this solution was applied.

The following gradient was used :

> t = 0 (min) 55%A ; 45%B
> t = 15 (min) 55%A; 45%B
> t = 70 (min) 0%A ; 100%B
> t = 75 (min) O%A; 100%B
> t = 76 (min) 55%A ; 45%B

Peaks are assigned to di- tri-and tetra- antennary, using Electrospray mass spectrometry (ESMS) and Matrix Assisted Laser Desorption Ionisation Time-Of Flight mass spectrometry (MALDI-TOF MS).

Results are expressed as relative percentages P of di-antennary ; tri-antennary and tetra-antennary, with 100% being the sum of all glycans. The AI is then calculated using the following equation:

$$AI \quad \simeq \quad 2\,P_{di} \quad + \quad 3\,P_{tri} \quad + \quad 4\,P_{tetra}$$

wherein AI is antennarity index, and $P_{di}$, $P_{tri}$ and $P_{tetra}$ are the percentage of total carbohydrate that is di-, tri- and tetra-branched respectively.

### Example 3

### Separation of FSH into fractions based on degree of sialylation

Recombinant FSH was separated into acidic and basic fractions using anion exchange chromatography on DEAE-Sepharose FF.

- The column used was ⌀ 1.6 x 20 cm (XK Pharmacia or equivalent) for laboratory scale purification (approximately 60 mg bulk protein), and 0 3.4 x 40 cm (Vantadge Amicon or equivalent) for larger scale purifications, packed with DEAE -Sepharose FF resin;
- The mobile phase had a flow rate of 150-250 cm/hour

    Equilibration buffer 1: 2M Tris-HCl pH $7.0\pm0.1$ ;
    Equilibration buffer 2: 25 mM Tris-HCl pH $7.0\pm0.1$, conductivity $2.15\pm1.5$ mS/cm;
    Elution buffer 1: 25mM Tris pH $7.0\pm0.1$, 35 mM NaCl, conductivity $5.8\pm0.4$ mS/cm (This buffer elutes the more basic isoforms.);
    Elution buffer 2: 25mM Tris pH $7.0\pm0.1$, 150mM NaCl, conductivity $18.3\pm0.5$ mS/cm (This buffer elutes the more acidic isoforms.);
    Regeneration solution: 0.5M NaOH, M NaCl
    Storage solution: 10 mM NaOH

- The column was maintained at $23\pm3°C°$ or $5\pm3$ °C

The FSH was prepared for loading on the column as follows:

Frozen rhFSH bulk was thawed at $5\pm3°C$. After thawing was complete, the solution (3-4 mg of rhFSH, as estimated by optical density at 276.4 nm, per ml of resin) was diluted with 2M Tris-HCl pH $7.0\pm0.1$ in the following ratio: 1 part of buffer and 79 parts of rhFSH bulk. The final concentration of tris-HCl was 25 mM. The pH was adjusted to $7.0\pm0.1$ with HCl 1M.

The column was prepared by flushing with 3 bed volumes (BV) of NaOH 0.5 M followed by 6 BV of water. Equilibration was carried out by flushing with 4-5 BV of Equilibration Buffer 1, until a pH of approximately 7 was measured. Flushing was then continued with 7-8 BV of Equilibration Buffer 2.

An rhFSH sample, prepared as above was loaded onto the column. After loading was completed, the column was flushed with 3 BV of Equilibration Buffer 1.

Elution with Elution Buffer 1 was then started and collection of the basic fraction was begun when the absorbance value (276.4 nm) started to rise, and was continued for $20\pm1$ bed volumes. The eluent was then changed to Elution Buffer 2, and collection of the acidic fraction was started as soon as the absorbance value (276.4 nm) started to rise, and was continued for $3\pm1$ bed volumes.

The fractions were then subjected to ultrafiltration, in order to concentrate them, with an ultrafiltration cell type 8400 (Amicon or equivalent) equipped with a YM3 membrane for the basic fraction and with a YM10 for the acidic fraction. All the operations were performed at $5\pm3°C$.

### Example 4

### Clinical study comparing FSH isoforms

The comparative efficacy on volunteers of two experimental rhFSH batches was assessed.

Two FSH preparations were generated by splitting rhFSH in two fractions, using ion-exchange chromatography, as described above, in Example 3. Batch A was deemed "acidic", and had a Z-number of 220 (i.e. acidic fraction from Example 3); while Batch B was deemed "basic" and had a Z-number of 160 (i.e. basic fraction from Example 3).

Using the conventional assay of ovarian weight increase in rats, ampoules of batch A and batch B were filled to contain approximately 150 IU FSH each.

The characteristics of the two batches are presented in Table 2. It should be noted that, since the vials were filled by IU, the actual amount of FSH in the ampoules of the basic Batch B was about 250% that of the acidic Batch A (about 24 $\mu$g Vs about 9 $\mu$g).

| Table 2: Characteristics of FSH Batches used in clinical study | | |
|---|---|---|
| | Batch A "acidic" | Batch B "basic" |
| FSH content per ampoule | 8.7 µg/ampoule | 23.8 µg/ampoule |
| Specific bioactivity | 19,753 IU/mg | 7,386 IU/mg |
| Z-number | 220 | 160 |
| Antennarity Index (AI) | 274 | 237 |

Specific bioactivity is calculated by dividing the activity in IU by the weight of protein.

The patient group was 32 pre-menopausal female volunteers. The patients were submitted to pituitary down-regulation by daily injections of decapeptyl (0.1 mg). After 14 days, a sonographic examination was performed and in the absence of cysts, stimulation was started with rFSH (150 IU/day) either Batch A or Batch B. Follicular growth was assessed by sonography and serum $E_2$ concentrations on a daily basis.

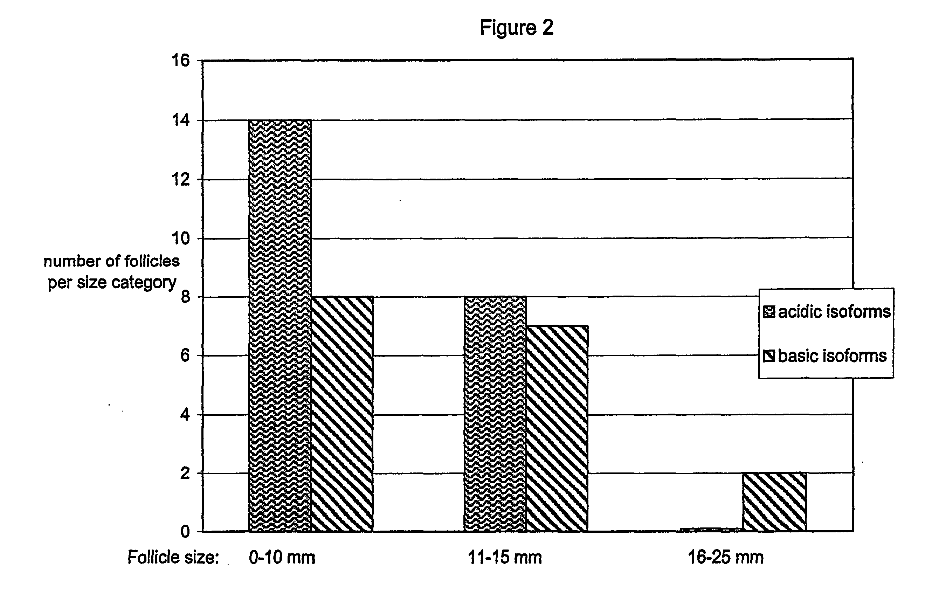

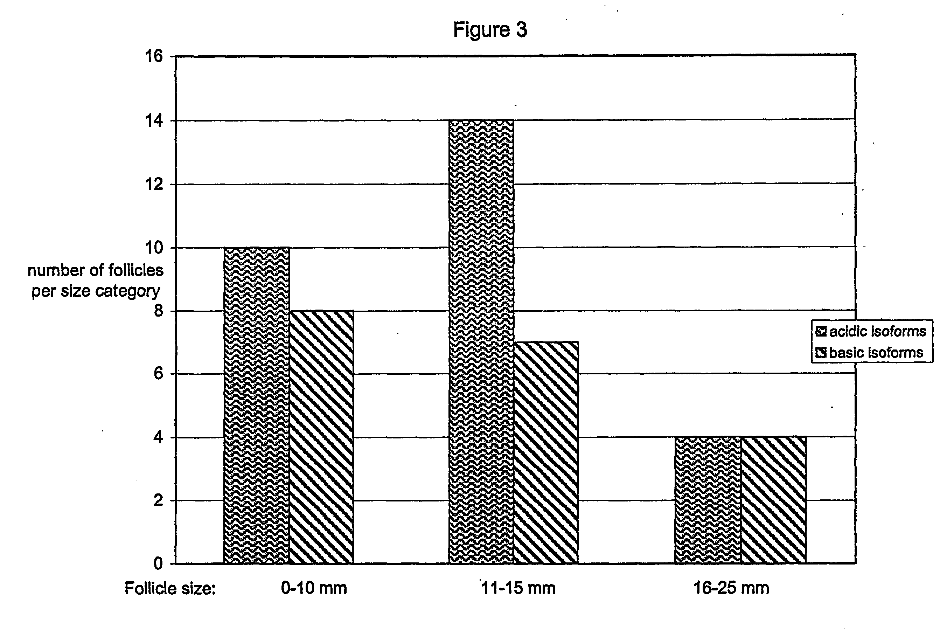

During FSH stimulation, follicles will develop and grow in diameter. The follicles of each patient were measured and counted on days 8 and 10 of stimulation, and the number of follicles falling into the size categories 0-10 mm, 11-15 mm and 16-25 mm were noted. In Figure 2, the average number of follicles per patient falling into each category is shown for patient groups treated with acidic and basic isoforms, on day 8. In Figure 3, the same plot is shown for day 10.

The results of the study showed that while in the basic group the follicle size progressed regularly with time, the acidic group gave rise to a second cohort of follicles, slightly delayed in respect to the first, with a consequent strong increase in the follicle formation, approximately doubling that in the basic group. This second cohort increased in size going from day 8 to day 10. The result is that in the patient group treated with "acidic" FSH, on day 10 there were on average a total of 18 follicles larger than 11 mm, whereas in the group treated with "basic" isoform, the average number of follicles larger than 11 mm on day 10 was only 11.

The average total number of follicles on day 10 in the "acidic" group is 28, whereas in the "basic" group it is 19.

The average total follicular volume (TFV) per patient was determined using sonography. TFV in the group receiving "acidic" FSH was 30% higher than that in the group receiving "basic" FSH.

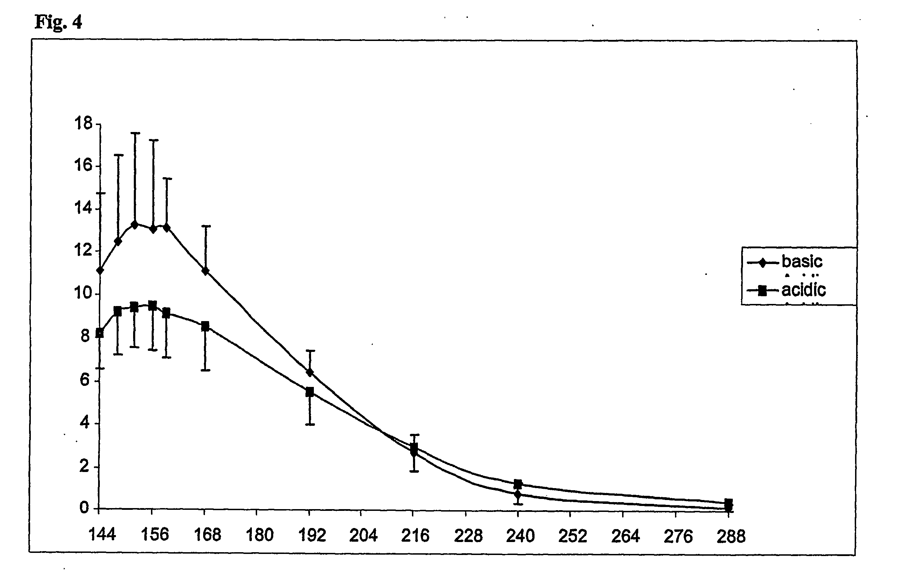

FSH serum levels in the patients, measured by radioimmunoassay, were higher in the basic group, as expected from the protein mass injected; however the difference was only about 30% (see Figure 4), as compared with the administration difference of 250%, in line with the higher metabolic endurance of the acidic forms.

## Example 5

### Separation of FSH into fractions based on Antennarity Index (AI)

FSH preparations having higher than normal antennarity indices can be isolated using HPLC or affinity chromatography with concanavalin A (Con-A) derivatised Sepharose.

## Example 6

### "Sialyl boosting" with sialyl transferase

Recombinant human FSH ("starting material"; 10 mg) was dissolved in buffer (0.1 M HEPES, pH 7.5) at a concentration of 4.3 mg/ml. To this solution was added recombinant rat sialyl transferase (ST3GalIII) to a concentration of 100 mU/ml, and cytidine-5'-monophosphate-N-acetyl neuraminic acid (CMP-NeuAc) as sialic acid donor at a concentration of 20 mM. Alternatively, the sialic acid donor may be generated *in situ* using 20 mM NeuAc and 2 mM CMP in the presence of CMP-sialic acid synthetase. The reaction was incubated at 37°C for 24 hours. Fractions enriched in sialic acid were isolated using the techniques described in Example 3.

Sialyl boosting may also be carried out using starting material consisting of FSH having an enhanced Antennarity Index, prepared according to Example 5.

Alternatively, sialyl boosting may be carried out with an FSH starting material already having an elevated Z-number, as compared with conventional recombinant FSH. Such starting material may be isolated using the techniques of Example 3.

**Example 7**

**Generation of FSH mutants**

The cDNAs of the α- and β-subunits of human FSH were subcloned into the pDONR vector (Invitrogen). The QuikChange™ Site-Directed Mutagenesis Kit (Stratagene) was used to introduce N-linked glycosylation sites into the α- and β-subunits of FSH. The QuikChange™ system utilises two synthetic oligonucleotide primers containing the desired mutation(s). The following pairs of oligonucleotides were used to introduce the N-linked glycosylation sites: CC TTG TAT ACA TAC CCA AAC GCC ACC CAG TGT CAC and GTG ACA CTG GGT GGC GTT TGG GTA TGT ATA CAA GG for V78N, GC TGT GCT CAC CAT AAC GAT TCC TTG TAT ACA TAC C and GGT ATG TAT ACA AGG AAT CGT TAT GGT GAG CAC AGC for A70N, GAT CTG GTG TAT AAG AAC CCA ACT AGG CCC AAA ATC CA and TGG ATT TTG GGC CTA GTT GGG TTC TTA TAC ACC AGA TC for D41 N/A43T, TGT ACT GTG CGA GGC CTG AAC CCC AGC TAC TGC TCC and GGA GCA GTA GCT GGG GTT CAG GCC TCG CAC AGT ACA for G100N, G AAC GTC ACC TCA AAC TCC ACT TGC TG and CA GCA AGT GGA GTT TGA GGT GAC GTT C for E56N, and CAG GAA AAC CCA ACC TTC TCC CAG CC and GG CTG GGA GAA GGT TGG GTT TTC CTG for F17T. The DNA sequences of the mutant cDNAs were confirmed using the ABI PRISM BigDye™ Terminator v3.0 Ready Reaction Cycle Sequencing Kit followed by analysis with the ABI PRISM 310 Genetic Analyzer.

The pCI mammalian expression vector (Promega) was converted into a GATEWAY destination vector by using the GATEWAY Vector Conversion System (Invitrogen). The α- and β-mutants along with the wild-type subunits were subcloned into the pCI expression vector using the Gateway™ Cloning Technology (Invitrogen). The pCI expression vector contains the human cytomegalovirus immediate-early enhancer/promoter to regulate the expression of the inserted gene, an intron upstream of the gene to promote expression and the simian virus 40 late polyadenylation signal downstream from the inserted gene to terminate transcription. The E56N and F17T alpha mutants in pCI were co-transfected with wild-type FSH β in pCI whereas the A70N, G100N, V78N and D41 N/A43T β-mutants in pCI were co-transfected with wild-type α-subunit in pCI. As a control, the wild-type β-subunit of FSH in pCI and the α-subunit in pCI were co-transfected. The plasmids were transiently transfected into HEK293 cells (ATTC, CRL-10852) using the calcium phosphate method (for example, as described in WO 96/07750). Alternatively, The pCI plasmid containing either the wild-type β-subunit or the V78N β-mutant was co-transfected with wild-type α-subunit in pCI. The plasmids may also be transiently or stably transfected into CHO cells. One day after the transfection the medium was changed to DMEM/F12 (Invitrogen, 11320-033) containing 1 ug/ml of insulin (Invitrogen, 18140-020), 6.8 ng/ml of sodium selenite (Sigma, S5261) and 12.2 ng/ml of ferric citrate (Sigma, F3388). One day following the change in the medium, the conditioned medium was collected and centrifuged for 5 min at approximately 800 x $g$ at 4°C to remove any cellular debris. The supernatant was removed and centrifuged at 16,000 x $g$ in a Biofuge fresco (Heraeus Instruments) for 5 minutes and then the medium was further clarified by filtering through a 0.45 $\mu$m Acrodisc filter (Gelman Sciences, 4184). To the clarified cellular extract was added, 1 M Tris, pH 7.4 for a final concentration of 50 mM Tris and Tween20 was added for a final concentration of 0.1% Tween20.

The FSH mutants were purified from the cellular extract using immuno-affinity chromatography, on Sepharose derivatised with anti-FSH monoclonal antibodies immobilised using divinyl sulfone (Immunoresin anti-FSH-McAb-DVS-Sepharose). Such resins can be produced by methods known to the skilled practitioner, for example, as disclosed in WO 88/10270.

The resin was equilibrated in equilibrating buffer, consisting of 0.1 M Tris-HCl, 0.3M NaCl buffer at pH=7.5, at 4°C. The column was loaded with a quantity of IU FSH (by radio-immunoassay, RIA) corresponding to 80-90% of the total FSH binding capacity of the column.

Non-retained proteins were eluted with equilibrating buffer (as above) until the $OD_{280}$ of the eluate was lower than 0.02.

The absorbed mutant FSH was eluted from the immunoresin with 1 M ammonia solution at 4°C. Eluates corresponding to about 4 times the immunoresin volume were pooled, the pH was adjusted to 9.0 by addition of glacial acetic acid at 4°C, as soon as possible after collection, and the solution was ultrafiltered in an Amicon apparatus (membrane cutoff 10,000 Da) and concentrated to a small volume.

The concentrated mutant FSH solution was then subjected to a step of reverse phase HPLC, using a Waters Prep LC 500A liquid chromotograph equipped with UV detector and a preparative gradient generator. Prior to application to the column, the pH of the solution was adjusted to about 5.6. The solution was loaded on a $C_{18}$ reversed phase column (Prepak 500 $C_{18}$ cartridges Waters) which had previously been equilibrated with 0.05 M ammonium acetate buffer pH=5.6 at room temperature. The flow rate was 100 ml/min and the eluate was monitored at 280 nm.

Mutant FSH was eluted by a gradient of isopropanol up to 50% of the mobile phase. Fractions were checked by analytical gas phase chromatography (GPC). and radioimmunoassay (RIA). The organic solvent was removed by distillation under vacuum at less than 40°C, and the solution was frozen and lyophilized.

The mutant FSH preparations expressed in CHO cells were subjected to ion exchange chromatography, as described in Example 3, in order to isolate fractions having $Z^+$-numbers of greater than 180, 190, 200, 210, 220, 230, 240, 250, and higher.

Mutant FSH preparations expressed in CHO or HEK293 cells were subjected to sialyl boosting, as described in Example 6. After sialyl boosting, the mutant FSH was subjected to ion exchange chromatography, according to Example 3, to isolate fractions having $Z^+$-numbers of greater than 180, 190, 200, 210, 220, 230, 240, 250, and higher.

**References:**

[1] Wide, L; *The regulation of metabolic clearance of human FSH in mice by variation of the molecular structure of the hormone; Acta Endocrinologica* **112** 1986; 336-344;

[2] Chappel *et al.; Endocr. Rev.* **4** *1983*; 179-211; Padmanabhan *et al.; J. Clin. Endocrinol. Met.* **67** *1988*; 465-473; Wide *et al.; J. Clin. Endocrinol. Met.* **70** *1990*, 271-276; Anobile *et al. Glycoform composition of serum gonadotropins through the normal menstrual cycle and in the postmenopausal state; Mol. Human Reproduct.* **4** *1998*; 631-639

[3] Morell *et al.; J. Biol. Chem.* **246** *1971*; 1461-1467; Ashwell *et al.; Annu. Rev. Biochem.* **51** *1982*; 531-554

[4] Steelman & Pohley; *Assay of the follicle stimulating hormone based on the augmentation with human chorionic gonadotropin; Endocrinology* **53** *1953*; 604-616

[5] D'Antonio *et al.; Biological characterisation of recombinant human follicle stimulating hormone* isoforms; *Human Reproduction* **14** *1999*; 1160-1167

[6] Vitt et *al.; Isoforms of human recombinant follicle-stimulating hormone: comparison of effects on murine follicle development* In Vitro; *Biol. Reproduct.* **59** *1998*; 854-861

[7] Timossi *et al.; Differential effects* of *the charge variants* of *human follicle-stimulating hormone; J. Endocrinol.* **165** *2000*; 193-205

[8] Zambrano *et al.; Studies on the relative in-vitro biological potency of the naturally-occurring isoforms of intrapituitary follicle stimulating hormone; Mol. Hum. Reprod.* **2** *1996*; 563-71

[9] Zambrano *et* al.; *Receptor binding activity and in vitro biological activity of the human FSH charge isoforms as disclosed by heterologous and homologous assay systems: implications for the structure-function relationship of the FSH variants; Endocrine* **10** *1999;* 113-121

[10] Wide et *al.; Influence of the assay method used on the selection of the most active forms of FSH from the human pituitary; Acta Endocrinol.* (Copenhagen) **113** 1986;17-22

[11] Mulders et *al.; Prediction of the in vivo biological activity of human recombinant follicle stimulating hormone using quantitative isoelectric focussing; Biologicals* **25** 1997; 269-281

[12] Timossi *et al.; A less acidic human follicle-stimulatig hormone preparation induces tissue-type plasminogen activator enzyme activity earlier than a predominantly acidic analogue in Phenobarbital-blocked pro-oestrous rats; Mol. Human Reproduct.* **4** *1998*; 1032-1038

[13] Healy *et al.; Lancet* **343** *1994*; 1539-1644

[14] for example, a technique is described in EP 0 170 502 (Serono Laboratories, Inc.)

[15] Buckler et *al.; Ovulation induction with low dose alternate day recombinant follicle stimulating hormone; Hum. Reprod.* **14** *1999*; 2969-73

[16] Hard et al.; *Isolation and structure determination of the intact sialylation N-linked carbohydrate chains of recombinant human follitropin expressed in Chinese hamseter ovary cells, Eur. J. Biochem.* *193* 1990; 263-271

[17] Nadano *et al.; J. Biol. Chem.* **261** *1986*; 11550-11557; Kanamori *et al.; J. Biol. Chem.* **265** 1990; 21811-21819

[18] Swedlow *at al.; Deglycosylation of gonadotropins with an endoglycosidase; Proc. Soc. Experiment. Biol. & Med.* **181** *1986*; 432-437

[19] Mulders *et al.; Biologicals* **25** *1997*; 269-281

[20] Zambrano *et al.; Mol. Hum. Reprod.* **2** *1996*; 563-571

[21] Timossi *et al.; Neuroendocrinology* **67** *1998*; 153-163

[22] Boime *et al.; Glycoprotein hormone structure-function and analog design; Recent Prog. Horm. Res.* **54** *1999*; 271-88

[23] Wen *et al.;* J. *Biol. Chem.* **267** *1992*; 21011; Van den Eijnden *et al. Enzymatic amplification involving glycosyltransferases forms* the *basis for the increased size of asparagine-linked glycans at the surface of NIH 3T3 cells expressing the N-ras proto-oncogene; J. Biol. Chem.* **266** *1991;* 21674

[24] Weinstein *et al. J. Biol. Chem.* 257 1982; 13845

25 Sasaki *et al. J. Biol. Chem.* 268 1993; 22782-22787; Kitagawa & Paulson; *J. Biol. Chem.* 269 1994;1394-1401

[26] Kitagawa *et al.; J. Biol. Chem.* **271** *1996;* 931-938

[27] for example, a conventional technique is described in EP 0 170 502 (Serono Laboratories, Inc.)

**SEQUENCE LISTING**

<120> Follicle stimulating hormones

<130>

<160> 2

<170> PatentIn version 3.0

<210> 1
<211> 92
<212> PRT
<213> Homo sapiens

<400> 1

```
Ala Pro Asp Val Gln Asp Cys Pro Glu Cys Thr Leu Gln Glu Asn Pro
1               5                   10                  15
Phe Phe Ser Gln Pro Gly Ala Pro Ile Leu Gln Cys Met Gly Cys Cys
                20                  25                  30
Phe Ser Arg Ala Tyr Pro Thr Pro Leu Arg Ser Lys Lys Thr Met Leu
        35                  40                  45
Val Gln Lys Asn Val Thr Ser Glu Ser Thr Cys Cys Val Ala Lys Ser
        50                  55                  60
Tyr Asn Arg Val Thr Val Met Gly Gly Phe Lys Val Glu Asn His Thr
65                  70                  75                  80
Ala Cys His Cys Ser Thr Cys Tyr Tyr His Lys Ser
                85                  90
```

<210> 2
<211> 111
<212> PRT
<213> Homo sapiens

<400> 2

Asn Ser Cys Glu Leu Thr Asn Ile Thr Ile Ala Ile Glu Lys Glu Glu
1                5                    10                  15
Cys Arg Phe Cys Ile Ser Ile Asn Thr Thr Trp Cys Ala Gly Tyr Cys
              20                  25                  30
Tyr Thr Arg Asp Leu Val Tyr Lys  Asp Pro Ala Arg Pro Lys Ile Gln
        35                    40                  45
Lys Thr Cys Thr Phe Lys Glu Leu Val Tyr Glu Thr Val Arg Val Pro
      50                  55                  60
Gly Cys Ala His His Ala Asp Ser Leu Tyr Thr Tyr Pro Val Ala Thr
65                  70                      75                  80
Gln Cys His Cys Gly Lys Cys Asp Ser Asp Ser Thr Asp Cys Thr Val
                    85                  90                  95
Arg Gly Leu Gly Pro Ser Tyr Cys Ser Phe Gly Glu Met Lys Glu
        100                  105                  110

**Claims**

1. A pharmaceutical composition comprising recombinant FSH, wherein the recombinant FSH has a Z-number that is at least at 200, preferably at least at 220, more preferably at least at 240, most preferably at least at 260.

2. The pharmaceutical composition of claim 1, for use in medicine.

3. The pharmaceutical composition according to claim 1 or 2 wherein the recombinant FSH has an antennarity index AI of from 220-280.

4. The pharmaceutical composition according to any of the preceding claims wherein the recombinant FSH is expressed in Chinese Hamster Ovary cells.

5. A use of recombinant FSH in the preparation of a medicament for use in folliculogenesis, wherein the recombinant FSH has a Z-number that is at least at 200.

6. A use according to claim 5 wherein the FSH is administered on a daily or semi-daily basis with a dose of 50-75 IU during less than 14 days.

7. A use of a recombinant FSH in the preparation of a medicament for use in controlled ovarian hyperstimulation, wherein the recombinant FSH has a Z-number that is at least at 200.

8. A use of a recombinant FSH in the preparation of a medicament for use in ovulation induction or intrauterine insemination, wherein the recombinant FSH has a Z-number that is at least at 200.

9. A use of a recombinant FSH in the preparation of a medicament for use in the treatment of male infertility, wherein the recombinant FSH has a Z-number that is at least at 200.

10. A use according to claim 9 wherein the FSH is administered at 3 times 150 to 300 IU per week until spermatogenesis reaches levels adequate for insemination.

11. The use according to any of claims 5 to 10, wherein the recombinant FSH has a Z-number that is at least at 220, preferably at least at 240, more preferably at least at 260.

12. A pharmaceutical composition or a use according to any of the preceding claims wherein the FSH has an average pl of less than 3.4, more preferably less than 3.3, particularly preferably less than 3.2.

13. A pharmaceutical composition or a use according to any of the preceding claims wherein the FSH has an increased degree of sialylation at one or more additional glycosylation sites present on an peptide appended to the FSH protein.

14. A pharmaceutical composition or a use according to claim 13 wherein the appended peptide is the carboxy terminal portion of the β-subunit of hCG.

15. A pharmaceutical composition or a use according to any of the preceding claims wherein the FSH has one or more mutations that introduce one or more additional glycosylation sites in the FSH protein and increase the degree of sialylation.

16. A pharmaceutical composition or a use according to claim 15 wherein the mutations are selected from one or more of A70N, V78N, G100N, D41 N/A43T in the β-subunit of FSH and /or one or more of F17T, E56N, H83N in the α-subunit of FSH.

17. A method for preparing a recombinant FSH preparation having a Z-number that is at least 200, the method comprising the step of reacting FSH with a sialic acid donor in the presence of 2,3-sialyltransferase.

18. The method of claim 17, wherein the recombinant FSH has a Z-number that is at least at 220, preferably at least at 240, more preferably at least at 260.

19. The method of any one of claims 17 or 18, wherein the sialic acid donor is CMP-sialic acid.

20. The method of any one of claims 17 to 19, wherein the sialyltransferase is rat ST3Gal III.

21. A method for preparing a recombinant FSH preparation having a Z-number that is at least at 200, the method comprising a step of isoelectric focussing, chromatofocussing or ion-exchange chromatography.

22. A method for preparing a recombinant FSH preparation having a Z-number that is at least at 200, the method comprising supplementing the culture medium in which the recombinant FSH is expressed with neuraminidase inhibitors and/or direct intracellular precursors for sialic acid synthesis, such as acetylmannosamine.

23. A method according to claim 21 or 22 wherein the recombinant FSH is expressed in Chinese Hamster Ovary cells.

**Patentansprüche**

1. Pharmazeutische Zusammensetzung, umfassend rekombinantes FSH, wobei das rekombinante FSH eine Z-Zahl aufweist, die bei mindestens 200 liegt, vorzugsweise bei mindestens 220, stärker bevorzugt bei mindestens 240, am meisten bevorzugt bei mindestens 260.

2. Pharmazeutische Zusammensetzung nach Anspruch 1 zur Verwendung in der Medizin.

3. Pharmazeutische Zusammensetzung gemäß Anspruch 1 oder 2, wobei das rekombinante FSH einen Antennaritätsindex AI von 220 - 280 aufweist.

4. Pharmazeutische Zusammensetzung gemäß einem der vorhergehenden Ansprüche, wobei das rekombinante FSH in Ovarialzellen des chinesischen Hamsters exprimiert wird.

5. Verwendung von rekombinantem FSH bei der Herstellung eines Arzneimittels zur Verwendung bei der Follikulogenese, wobei das rekombinante FSH eine Z-Zahl aufweist, die bei mindestens 200 liegt.

6. Verwendung gemäß Anspruch 5, wobei das FSH über weniger als 14 Tage mit einer Dosis von 50 - 75 IE auf täglicher oder halbtäglicher Basis verabreicht wird.

7. Verwendung eines rekombinanten FSH bei der Herstellung eines Arzneimittels zur Verwendung bei kontrollierter ovarieller Hyperstimulation, wobei das rekombinante FSH eine Z-Zahl aufweist, die bei mindestens 200 liegt.

8. Verwendung eines rekombinanten FSH bei der Herstellung eines Arzneimittels zur Verwendung bei der Ovulationsinduktion oder intrauterinen Insemination, wobei das rekombinante FSH eine Z-Zahl aufweist, die bei mindestens 200 liegt.

9. Verwendung eines rekombinanten FSH bei der Herstellung eines Arzneimittels zur Verwendung bei der Behandlung männlicher Unfruchtbarkeit, wobei das rekombinante FSH eine Z-Zahl aufweist, die bei mindestens 200 liegt.

10. Verwendung gemäß Anspruch 9, wobei das FSH mit 3-mal 150 bis 300 IE pro Woche verabreicht wird, bis die Spermatogenese Werte erreicht, die zur Insemination adäquat sind.

11. Verwendung gemäß einem der Ansprüche 5 bis 10, wobei das rekombinante FSH eine Z-Zahl aufweist, die bei mindestens 220, vorzugsweise bei mindestens 240, stärker bevorzugt bei mindestens 260 liegt.

12. Pharmazeutische Zusammensetzung oder Verwendung gemäß einem der vorhergehenden Ansprüche, wobei das FSH einen durchschnittlichen pl-Wert von weniger als 3,4, stärker bevorzugt weniger als 3,3, besonders bevorzugt weniger als 3,2 aufweist.

13. Pharmazeutische Zusammensetzung oder Verwendung gemäß einem der vorhergehenden Ansprüche, wobei das FSH an einer oder mehreren zusätzlichen Glykosylierungsstellen, die auf einem Peptid, das an das FSH-Protein angehängt ist, vorliegen, einen erhöhten Sialylierungsgrad aufweist.

14. Pharmazeutische Zusammensetzung oder Verwendung gemäß Anspruch 13, wobei es sich bei dem angehängten Peptid um den carboxyterminalen Anteil der β-Untereinheit von hCG handelt.

15. Pharmazeutische Zusammensetzung oder Verwendung gemäß einem der vorhergehenden Ansprüche, wobei das FSH eine oder mehrere Mutationen aufweist, die eine oder mehrere zusätzliche Glykosylierungsstellen in das FSH-Protein einführen und den Sialylierungsgrad erhöhen.

16. Pharmazeutische Zusammensetzung oder Verwendung gemäß Anspruch 15, wobei die Mutationen aus einer oder mehreren von A70N, V78N, G100N, D41 N/A43T in der β-Untereinheit von FSH und/oder einer oder mehreren von F17T, E56N, H83N in der α-Untereinheit von FSH ausgewählt sind.

17. Verfahren zum Herstellen einer rekombinanten FSH-Zubereitung mit einer Z-Zahl, die mindestens 200 beträgt, wobei das Verfahren den Schritt umfasst, FSH in Anwesenheit von 2,3-Sialyltransferase mit einem Sialinsäuredonator reagieren zu lassen.

18. Verfahren nach Anspruch 17, wobei das rekombinante FSH eine Z-Zahl aufweist, die bei mindestens 220, vorzugsweise bei mindestens 240, stärker bevorzugt bei mindestens 260 liegt.

19. Verfahren nach einem der Ansprüche 17 oder 18, wobei es sich bei dem Sialinsäuredonator um CMP-Sialinsäure handelt.

20. Verfahren nach einem der Ansprüche 17 bis 19, wobei es sich bei der Sialyltransferase um ST3 Gal III aus der Ratte handelt.

21. Verfahren zum Herstellen einer rekombinanten FSH-Zubereitung mit einer Z-Zahl, die bei mindestens 200 liegt, wobei das Verfahren einen isoelektrischen Fokussierungs-, Chromatofokussierungs- oder Ionenaustauschchromatographieschritt umfasst.

22. Verfahren zum Herstellen einer rekombinanten FSH-Zubereitung mit einer Z-Zahl, die bei mindestens 200 liegt, wobei das Verfahren das Ergänzen des Kulturmediums, in dem das rekombinante FSH exprimiert wird, mit Neuraminidase-Inhibitoren und/oder direkten intrazellulären Vorläufern für die Sialinsäuresynthese, wie zum Beispiel Acetylmannosamin, umfasst.

23. Verfahren gemäß Anspruch 21 oder 22, wobei das rekombinante FSH in Ovarialzellen des chinesischen Hamsters exprimiert wird.

**Revendications**

1. Composition pharmaceutique comprenant une FSH recombinante, ayant un nombre Z qui est au moins de 200, de préférence au moins de 220, plus préférablement au moins de 240, et plus préférablement encore au moins de 260.

2. Composition pharmaceutique selon la revendication 1, pour son utilisation en médecine.

3. Composition pharmaceutique selon l'une quelconque des revendications 1 ou 2, dans laquelle la FSH recombinante a un taux de ramification A1 de 220 à 280.

4. Composition pharmaceutique selon l'une quelconque des revendications précédentes, dans laquelle la FSH recombinante est exprimée dans des cellules CHO (Chinese Hamsters Ovary Cells).

5. Utilisation de FSH recombinante dans la préparation d'un médicament pour son utilisation en folliculogénèse, dans laquelle la FSH recombinante a un nombre Z qui est au moins de 200.

6. Utilisation selon la revendication 5, dans laquelle la FSH est administrée sur une base journalière ou semi- journalière à une dose de 50 à 75 UI pendant moins de 14 jours.

7. Utilisation de FSH recombinante dans la préparation d'un médicament pour son utilisation dans l'hyperstimulation ovarienne contrôlée, dans laquelle la FSH recombinante a un nombre Z qui est au moins de 200.

8. Utilisation de FSH recombinante dans la préparation d'un médicament pour son utilisation dans l'induction de l'ovulation ou l'insémination intra-utérine, dans laquelle la FSH recombinante a un nombre Z qui est au moins de 200.

9. Utilisation de FSH recombinante dans la préparation d'un médicament pour son utilisation dans le traitement de l'infertilité mâle, dans laquelle la FSH recombinante a un nombre Z qui est au moins de 200.

10. Utilisation selon la revendication 9, dans laquelle la FSH est administrée 3 fois par semaine à une concentration de 150 à 300 UI jusqu'à ce que la spermatogenèse atteigne des niveaux adéquats pour l'insémination.

11. Utilisation selon l'une quelconque des revendications 5 à 10, dans laquelle la FSH recombinante a un nombre Z qui est au moins de 220, de préférence au moins de 240, et plus préférablement encore au moins de 260.

12. Composition pharmaceutique ou utilisation selon l'une quelconque des revendications précédentes, dans laquelle la FSH a un pI moyen de moins de 3,4, plus préférablement de moins de 3,3, de manière particulièrement préférée de moins de 3,2.

13. Composition pharmaceutique ou utilisation selon l'une quelconque des revendications précédentes, dans laquelle la FSH a un degré augmenté de sialylation à un ou plusieurs sites de glycosylation supplémentaires présents sur un peptide annexé à la protéine FSH.

14. Composition pharmaceutique ou utilisation selon la revendication 13, dans laquelle le peptide annexé est une portion carboxy-terminale de la sous-unité β de hCG.

15. Composition pharmaceutique ou utilisation selon l'une quelconque des revendications précédentes, dans laquelle la FSH a une ou plusieurs mutations qui introduisent un ou plusieurs sites de glycosylation supplémentaires dans la protéine FSH et augmente le degré de sialylation.

16. Composition pharmaceutique ou utilisation selon la revendication 15, dans laquelle la ou les mutations sont choisies parmi A70N, V78N, G100N, D41N/A43T dans la sous-unité β de FSH et/ou parmi F17T, E56N, H83N dans la sous-unité α de FSH.

17. Procédé d'une préparation de FSH recombinante ayant un nombre Z qui est au moins de 200, procédé comprenant l'étape de faire réagir la FSH avec un donneur d'acide sialique en présence de la 2,3-sialyltransférase.

18. Procédé selon la revendication 17, dans laquelle la FSH recombinante a un nombre Z qui est au moins de 220, de préférence au moins de 240, plus préférablement au moins de 260.

**19.** Procédé selon l'une quelconque des revendications 17 ou 18, dans lequel le donneur d'acide sialique est l'acide CMP-sialique.

**20.** Procédé selon l'une quelconque des revendications 17 à 19, dans lequel la sialyltransférase est la ST3Gal III de rat.

**21.** Procédé d'une préparation de FSH recombinante ayant un nombre Z qui est au moins de 200, procédé comprenant une étape de focalisation isoélectrique, focalisation chromatographique ou chromatographie échangeuse d'ions.

**22.** Procédé pour une préparation de FSH recombinante ayant un nombre Z qui est au moins de 200, procédé comprenant le fait d'ajouter à un milieu de culture dans lequel la FSH recombinante est exprimée, des inhibiteurs de neuraminidase et/ou des précurseurs intracellulaires directs de la synthèse d'acide sialique, tels que l' acéthylmannosamine.

**23.** Procédé selon l'une des revendications 21 ou 22, dans lequel la FSH recombinante est exprimée dans des cellules CHO (Chinese Hamsters Ovary Cells).

Fig. 1

## Figure 2

Figure 2 — Bar chart of number of follicles per size category versus follicle size (0-10 mm, 11-15 mm, 16-25 mm) for acidic isoforms and basic isoforms.

## Figure 3

Figure 3

Chart showing number of follicles per size category (y-axis, 0–16) by follicle size categories (x-axis: 0-10 mm, 11-15 mm, 16-25 mm), with bars for acidic isoforms and basic isoforms.

Legend: acidic isoforms; basic isoforms

Follicle size: 0-10 mm; 11-15 mm; 16-25 mm

EP 1 438 336 B1

Fig. 4

EP 1 438 336 B1

## Figure 5

Ala Pro Asp Val Gln Asp Cys Pro Glu Cys Thr Leu Gln Glu Asn Pro
1 5 10 15

Phe Phe Ser Gln Pro Gly Ala Pro Ile Leu Gln Cys Met Gly Cys Cys
20 25 30

Phe Ser Arg Ala Tyr Pro Thr Pro Leu Arg Ser Lys Lys Thr Met Leu
35 40 45

Val Gln Lys Asn Val Thr Ser Glu Ser Thr Cys Cys Val Ala Lys Ser
50 55 60

Tyr Asn Arg Val Thr Val Met Gly Gly Phe Lys Val Glu Asn His Thr
65 70 75 80

Ala Cys His Cys Ser Thr Cys Tyr Tyr His Lys Ser
85 90

EP 1 438 336 B1

## Figure 6

Asn Ser Cys Glu Leu Thr Asn Ile Thr Ile Ala Ile Glu Lys Glu Glu

Cys Arg Phe Cys Ile Ser Ile Asn Thr Thr Trp Cys Ala Gly Tyr Cys

Tyr Thr Arg Asp Leu Val Tyr Lys  Asp Pro Ala Arg Pro Lys Ile Gln

Lys Thr Cys Thr Phe Lys Glu Leu Val Tyr Glu Thr Val Arg Val Pro

Gly Cys Ala His His Ala Asp Ser Leu Tyr Thr Tyr Pro Val Ala Thr

Gln Cys His Cys Gly Lys Cys Asp Ser Asp Ser Thr Asp Cys Thr Val

Arg Gly Leu Gly Pro Ser Tyr Cys Ser Phe Gly Glu Met Lys Glu

EP 1 438 336 B1